# EUROPEAN PATENT SPECIFICATION

(11) **EP 1 401 493 B1**
(45) Date of publication and mention of the grant of the patent: **28.02.2007**
(21) Application number: 02743621.1
(22) Date of filing: 31.05.2002
(51) Int. Cl.: A61K 39/39, A61K 39/12, C12N 15/82, A01H 5/00, A61P 31/00

(54) **SUBUNIT VACCINES AND PROCESSES FOR THE PRODUCTION THEREOF**
UNTEREIHEITSIMPFSTOFFE UND VERFAHREN ZUR DEREN HERSTELLUNG
VACCINS SOUS-UNITES ET LEURS PROCEDES DE PRODUCTION

(30) Priority: 11.06.2001 IT RM20010332
(43) Date of publication of application: 31.03.2004
(73) Proprietor: Ente per le Nuove Tecnologie, l'Energia e l'Ambiente - ENEA, 00196 Rome (IT); Istituto Superiore di Sanità, 00161 Roma (IT); ISTITUTI FISIOTERAPICI OSPITALIERI (IFO) ISTITUTO REGINA ELENE, 00128 Rome (IT)
(72) Inventor: FRANCONI, Rosella, I-00061 Anguillara Sabazia RM (IT); GIORGI, Colomba, I-00192 Roma (IT); VENUTI, Aldo, I- 00153 Roma (IT); MARCANTE, Maria-Luisa, I-00191 Roma (IT); ACCARDI, Luisa, I- 00135 Roma (IT); DI BONITO, Paola, I-00175-Roma (IT); DIBELLO, Francesco, I-00061 Anguillara Sabazia RM (IT); BITTI, Orsola, I-01010 Veiano VT (IT)
(74) Representative: Germinario, Claudio
(86) International application number: PCT/IT2002/000354
(87) International publication number: WO 2002/102410

(56) References cited:
- WO-A-01/07613
- WO-A-01/61022
- WO-A-01/66778
- WO-A-96/12801
- GERARD C M ET AL: "Therapeutic potential of protein and adjuvant vaccinations on tumour growth" VACCINE, BUTTERWORTH SCIENTIFIC. GUILDFORD, GB, vol. 19, no. 17-19, 21 March 2001 (2001-03-21), pages 2583-2589, XP004231084 ISSN: 0264-410X
- KOPROWSKI H ET AL: "The green revolution: plants as heterologous expression vectors" VACCINE, BUTTERWORTH SCIENTIFIC. GUILDFORD, GB, vol. 19, no. 17-19, 21 March 2001 (2001-03-21), pages 2735-2741, XP004231105 ISSN: 0264-410X

## Description

### Field of the Invention

The present invention refers to the field of immunology and in particular to the sector of the so-called subunit vaccines.

### State of the Art

Subunit vaccines are known to the art.

Those are vaccines comprising as immunogen specific macromolecules (vaccinogens) able to induce a protective immune response towards a certain pathogen, or a certain tumour-associated antigen. In particular, among the vaccinogens used in the art, there are molecules of protein nature derived from pathogens, like, e.g., viruses or bacteria, per se not able to determine the onset of the illness.

Therefore, such vaccines allow vaccination strategies which are safer with respect to those proper of the traditional vaccines, based on whole, killed or inactivated pathogens.

However, with respect to the former, the subunit vaccines present a first problem resulting from the fact that the vaccinogens used in the art are molecules that per se generally exhibit scarce or even absent immunogenicity.

In order to overcome this problem, the subunit vaccines often comprise, or are administered along with, adjuvants, i.e., substances able to increase the immunogenicity of the utilized vaccinogen when administered therewith, whose action mechanisms are still not completely known.

Among all known adjuvants, the aluminum-based mineral salts (alum) and the MF59 are the only ones demonstrated to be safe enough to be approved for human use. However,' aluminum is a weak adjuvant for antibody induction and for cell-mediated immunity response in the case of subunit protein-based vaccines.

Research on adjuvants is presently very active, also due to the development of new-generation vaccines (e.g. recombinant and mucosal vaccines). For example, prior document WO-A-9612801 discloses the use of a partially purified extract of soluble proteins from a tobaco plant transformed with vectors encoding antigens and its a use in a vaccine composition.

The effectiveness of these adjuvants is evaluated from the presence of helper and cytotoxic lymphocyte clones, from the presence of some immunoglobulin classes (e.g. IgG₂) and of Th1-type cytokines, upon administration of vaccines containing them. Particularly effective are considered the adjuvants able to elicit a humoral as well as a cell-mediated response (like, e.g., the Quil-A, at least in the experimental animal).

Additionally to adjuvant effectiveness, a further relevant parameter in the study and development of the adjuvants is that of their safety. In clinical trials, many experimental adjuvants are used. Though some of the latter could be employed in those applications (like anti-cancer therapy) in which a certain level of side effects is tolerable, most of them are too toxic for routine clinical use.

A second problem of the subunit vaccines is that of their storage , particularly in all those cases in which the vaccinogens are molecules of protein nature, hence exhibiting all the problems related to the storage of purified proteins with the related instability and thermolability of the vaccine compositions.

A third problem of the subunit vaccines is related to their production in industrially relevant quantities. Once the vaccinogen is identified, the process leading to its production generally comprises the isolation of the corresponding gene and the its expression in a heterologous system. However, the systems used in the art do not always allow to produce the vaccinogen in native form, and often entail low yields and rapid degradation, with generally lengthy and costly purification procedures.

Subunit vaccines representative of the problems highlighted above are those comprising the E7 protein of the human papillomavirus (HPV), per se scarcely immunogenic and highly unstable.

In fact, the HPV E7 protein exhibits a low half-life in vivo (about 30-40 min, after 1 hour about 70% of the protein is degraded) (Reinstein E, Scheffner M, Oren M et al (2000). 'Degradation of the E7 human papillomavirus oncoprotein by the ubiquitin-proteasome system: targeting via ubiquitination of the N-terminal residue'. *Oncogene* 19: 5944-5950). The instability also characterises the recombinant E7 protein purified from eukaryotic expression systems (yeast, insect) or from bacteria, used in clinical trials of therapeutic vaccines.

Attempts at purifying large quantities of E7 protein not fused to other proteins or peptides led to very low yields, due to a rapid proteolytic degradation.

### Summary of the invention

Object of the present invention is to provide subunit vaccines allowing to overcome at least one of the drawbacks highlighted above and therefore be immunogenic, stable and/or produced by procedures quicker and simpler with respect to those proper of the state of the art.

According a first object of the invention is the use of an extract in buffer without detergent and denaturing substances of soluble proteins from leaf cells of a plant selected from the group comprising *Nicotiana benthamiana, Nicotiana tabacum* and *Chenopodium quinoa,* preferably *Nicotiana benthamiana,* for the preparation of an adjuvant composition to be used with a subunit vaccines comprising this extract as adjuvant along with a vaccinogen of interest. A second object is the subunit vaccines comprising said extract.

Actually, the main advantage of the subunit vaccines of the invention ensues from the ability of the foliar cell extract of acting as adjuvant. In fact, this extract is able to increase the immunogenicity of a vaccinogen of interest, until enabling the latter to induce immune response of a humoral as well as a cell-mediated immune response, in a way in all comparable to that of adjuvants like the Quil-A. This is so particularly in the case of vaccinogens consisting of antigens of viral nature like the E7 protein of the human papillomavirus (HPV).

Consequently to the adjuvanting ability of the abovementioned extract, the resulting vaccine can be administered even without added adjuvant and it can protect against tumour development in animal models.

The foliar cell extract of *Nicotiana benthamiana, Nicotiana tabacum* and *Chenopodium quinoa,* can be obtained according to the invention by adopting the extraction procedures known to a person skilled in the art and determinable by the former on the basis of the information provided in the present patent application and in the documents cited therein. However, it has to be highlighted that to the ends of the invention the use in the extraction procedures and, accordingly, the presence of protease inhibitors in the extract, does not affect the functionalities of the extract_itself.

A person skilled in the art will also be aware of the modes of combining the vaccinogen, in a preferred embodiment consisting of the HPV E7 protein, and the extract in the same vaccine composition. The skilled person may single out said modes in the light of what is reported in the present application and in the cited documents.

In a particularly preferred embodiment, said combining is carried out by means of a process for producing a subunit vaccine, it also object of the invention, comprising the steps of:
a. expressing in soluble form a protein antigen in leaf cells of a plant selected from a group comprising *Nicotiana benthamiana, Nicotiana tabacum* and *Chenopodium quinoa,* preferably *Nicotiana benthamiana,* at a concentration greater than or equal to 3 µg of protein per gram of leaf; and
b. preparing an extract in buffer without detergent and denaturing substances of total soluble proteins from said cells; said plant extract being useful as subunit vaccine.
   A first advantage associated to this process lies in the possibility to obtain thereby the vaccinogen in native form comprised in extracts directly useful to immunize an animal or a human being.
   A second advantage of the process of the invention lies in that said vaccinogen-containing extract can be obtained in quantities suitable to the administration for immunogenic purposes within a very short time (1-2 weeks).
   A third advantage of the invention lies in that such extract is able to elicit a Th1-type and a Th2-type immune response, hence being useful for both prophylactic and therapeutic vaccines.
   A further advantage of the invention lies in that the antigen-containing plants of *Nicotiana benthamiana, Nicotiana tabacum* and *Chenopodium quinoa,* (in particular those of *Nicotiana benthamiana*)*,* also object of the invention as obtainable by the above step a., can be stored at room temperature, whereas the vaccine composition comprising said extract is stable at +4°C.
   In a preferred embodiment, the concentration of which at step a. is reached by transfection with a plant vector, in particular with a viral vector which in a more preferred embodiment is derived from the potato virus X (PVX).
   Concerning step b., it should be carried out by passages finally yielding an extract free from substances injurious to the organism to which the vaccine is destined.
   Thus, step b. is advantageously carried out using techniques known in the art to extract total soluble proteins directly in the absence of detergents and of denaturing substances, determinable by a person skilled in the art in the light of the present application and of the documents cited herein. However, as abovementioned, the use of protease inhibitors is unnecessary to the ends of the invention.
   Through the process of the invention extracts can be produced comprising any one vaccinogen of protein nature of known nucleotide and/or amino acid sequence.
   Among the preferred vaccinogens there are those consisting of a viral antigen, which in a particularly preferred embodiment is provided by the E7 protein of the HPV virus. In fact, this embodiment is particularly preferred in the light of the scarce immunogenicity and of the instability exhibited by said protein per se in the absence of adjuvants.
   Thus, the vaccine produced according to the abovedescribed process of the invention constitutes a preferred embodiment of the subunit vaccine of the invention. This is so since the vaccinogens comprised in the vaccine, and in particular the vaccinogen of viral nature like the E7 protein of the HPV16, perform their full immunogenic action above all when synthetized by the plant from which the extract is prepared.
   In the light of the above, object of the invention is also the use of the vaccine of the invention for the treatment of pathologies for the therapy of which it is necessary or advisable to induce an immune response of a humoral as well as of a cell-mediated type towards a determined antigen, such as, in particular, infections from viruses, bacteria or other microrganisms and from tumour-associated antigens.
   The ability to induce a cell-mediated response renders the use of the vaccinal preparation of the invention particularly expedient in the treatment of all those pathologies whose therapy requires a modification of the immune response, such as tumours or autoimmune diseases.
   Alternatively to the embodiment of the invention in which extract and vaccinogen are comprised in an individual vaccinal preparation prepared in any which way, the extract of the invention and the vaccinogen of interest (included in a composition comprising the vaccinogen and the compatible adjuvants) can be comprised in a kit for the treatment of the aboveindicated pathologies which comprises them for concomitant, sequential or separated use in the treatment of said pathologies.
   In particular, the vaccinogen comprised in said kit can be obtained according to the process disclosed hereinafter.
   In fact, according to a further aspect of the invention, the above described object is also attained by a process for the production of a vaccinogen comprising the steps a. and b. of the abovedescribed process and the further step of
c. purifying said vaccinogen from said extract.
   In fact, the vaccinogen thus produced can expediently be used for the preparation of a vaccine for the treatment of a pathology associated to the accumulation of the antigen corresponding to said vaccinogen.
   In this embodiment the vaccinogen should anyhow be administered along with an adjuvant, though its use upon purification from plant extracts provides procedures which are simpler, faster and safer with respect to those known in the art for vaccinogen production.
   Moreover, the vaccinogen thus produced is advantageously expressed in its entirety, hence providing all of its antigenic determinants, at high levels and in a stable form in plant extracts stored at 4°C for more than 10 days.

In this case, the outcome of the purification process followed by the process for the production of sera in animals, leads to the production of hyperimmune sera against gene products of which a mere fraction of the nucleotide sequence and not the natural protein is known.

Moreover, the abovementioned production procedure provides remarkable features of stability.

Hereinafter, the invention will be described making reference to the attached Figures.

### Description of the Figures

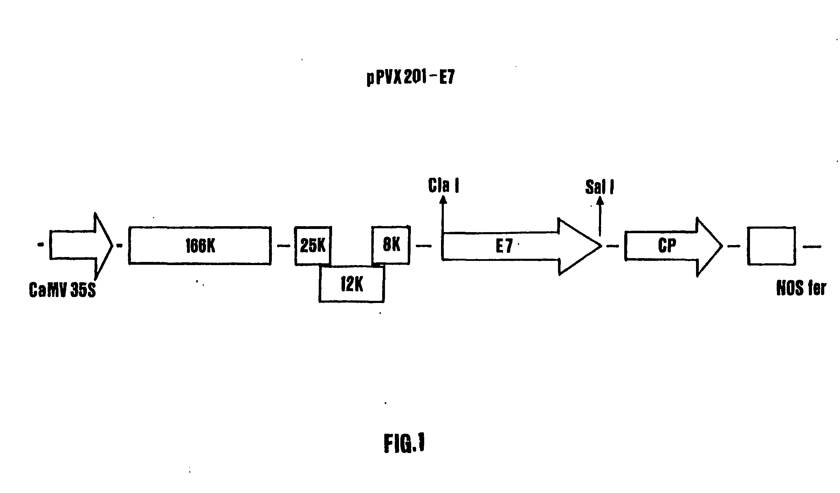

Figure 1 is a schematic representation of the vector pPVX201-E7, derived from PVX genome, used for the transient expression of the gene encoding the E7 oncoprotein of the human papillomavirus 16 (HPV16) in plants.

The term CaMV35S indicates the promoter 35S of the cauliflower mosaic virus; the term 166 K indicates the gene encoding the protein required for PVX replication; the terms 25 K, 12 K, 8 K indicate the three partially overlapped 'open reading frames', coding for the 25, 12 and 8 KDa PVX proteins, respectively; the term CP indicates the gene for the PVX coat protein; the term NOS-ter indicates the transcription termination signal derived from the nopaline synthase gene of *Agrobacterium tumefaciens;* the terms ClaI, SalI, indicate the restriction enzymes cleavage sites located at the cloning sites of the heterologous gene.

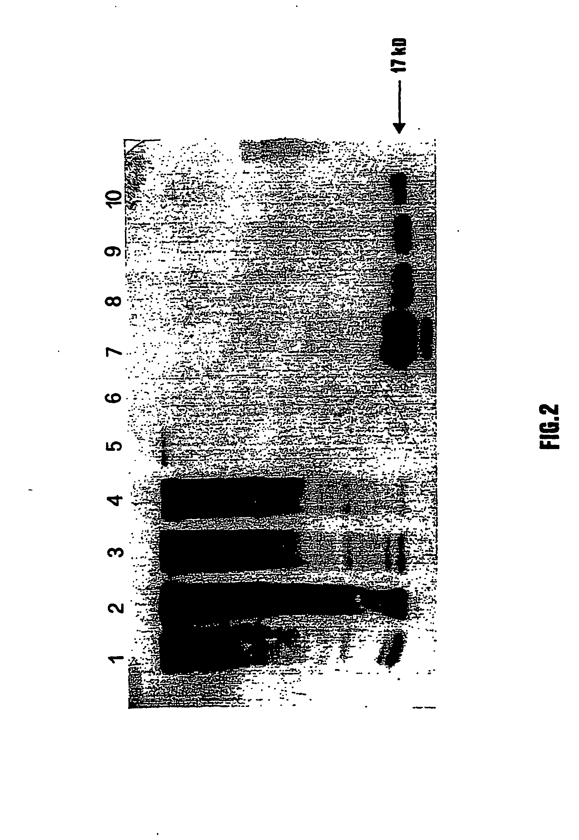

Figure 2 shows the result of an immunoblot (Western blot) assay of the proteins extracted from plants of *Nicotiana benthamiana* infected with PVX201-E7 (plasmid or virion, 7 days post-inoculation).

Numbers 1 and 2, indicate the lanes loaded, respectively, with 15 and 30 µg of total soluble proteins from unfiltered leaf extracts deriving from PVX201-E7 infected plants. Numbers 3 and 4, indicate the lanes loaded, respectively, with 15 and 30 µg of total soluble proteins from leaf extracts, filtered with a 0.22 µm filter, deriving from PVX201-E7 infected plants. Number 5 indicates the lane loaded with 20 µg of total soluble proteins from leaves of wild type PVX-infected plants. Number 6 indicates the lane loaded with the molecular weight marker for proteins. Numbers 7, 8, 9, and 10, indicate the lanes loaded, respectively, with 67, 40, 26, 13 ng of His-E7 protein purified by *E. coli.*

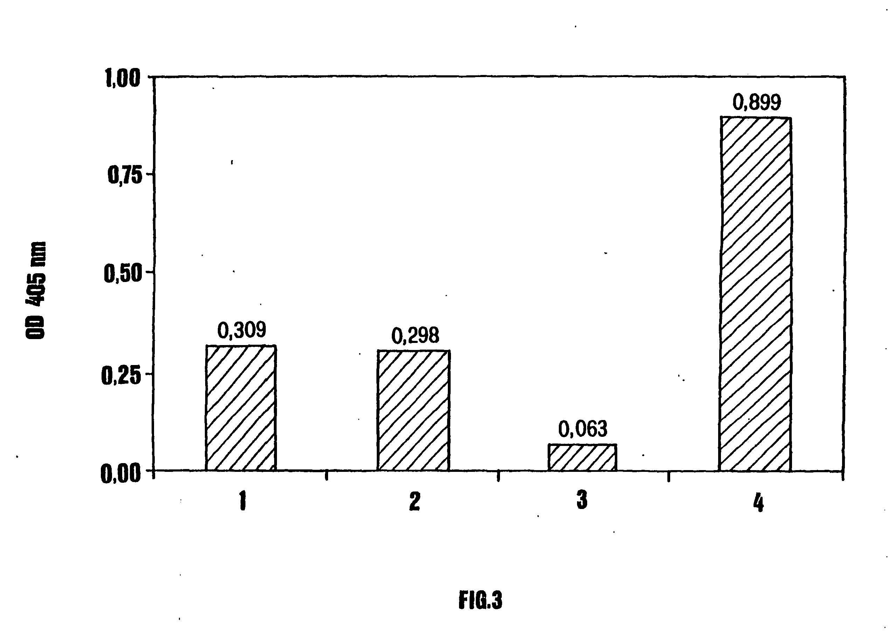

Figure 3 is a diagram illustrating the detection of the E7 protein in PVX201-E7-infected *N. benthamiana* plants by TAS ('triple antibody sandwich')-ELISA. On the y-axis the absorbance values are reported, whereas on the x-axis the extracts to which said values are referred are reported.

In particular, number 1 on the x-axis indicates the histogram related to extracts of PVX201-E7-infected plants; number 2 on the x-axis indicates the histogram related to extracts, filtered with a 0.22 µm filter, of PVX201-E7 infected plants; number 3 on the x-axis indicates the histogram related to extracts of wild type PVX201 infected plants; number 4 on the x-axis indicates the histogram related to 600 ng of His-E7 protein purified by E. coli.

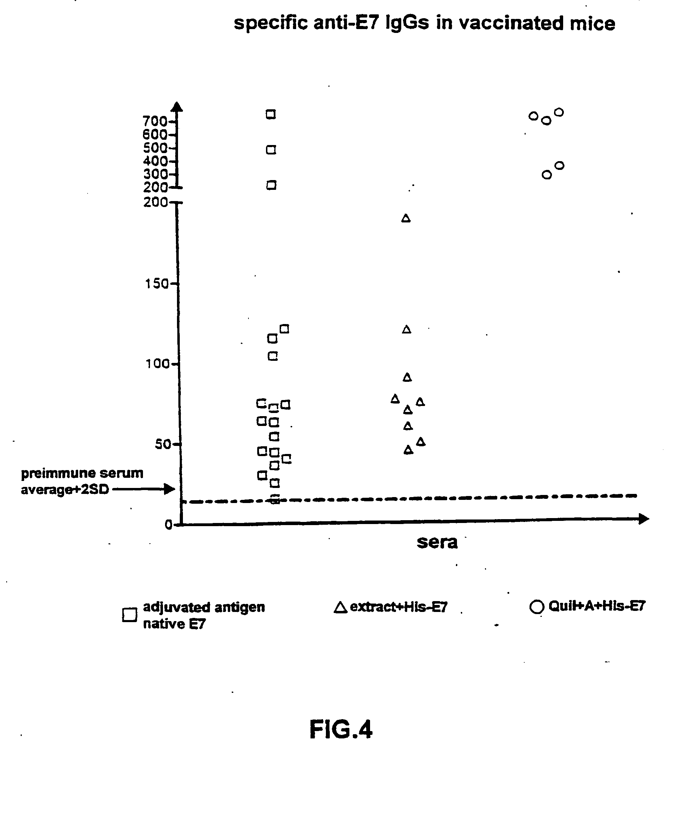

Figure 4 is a diagram illustrating the ELISA reactivity of sera of mice immunized with.the extracts of the invention. On the y-axis the relative reactivity of the anti-E7 IgG, and on the x-axis the proteins or the extracts to which said reactivity refers are reported.

In particular, the samples of sera of animals vaccinated with the plant extracts containing the native E7 protein are marked by the symbol □, the sera of animals vaccinated with foliar extract to which the recombinant His-E7 protein obtained in *E.coli* had been added is marked by the symbol ∇ and, finally, the control animals vaccinated with the recombinant His-E7 protein and the adjuvant Quil-A are marked by the symbol O. The value of the preimmunized sera increased of two times the standard deviation was taken as cutoff value of the sera and it is depicted as a broken line in the graph. All the animals were vaccinated subcutaneously with the same vaccination scheme.

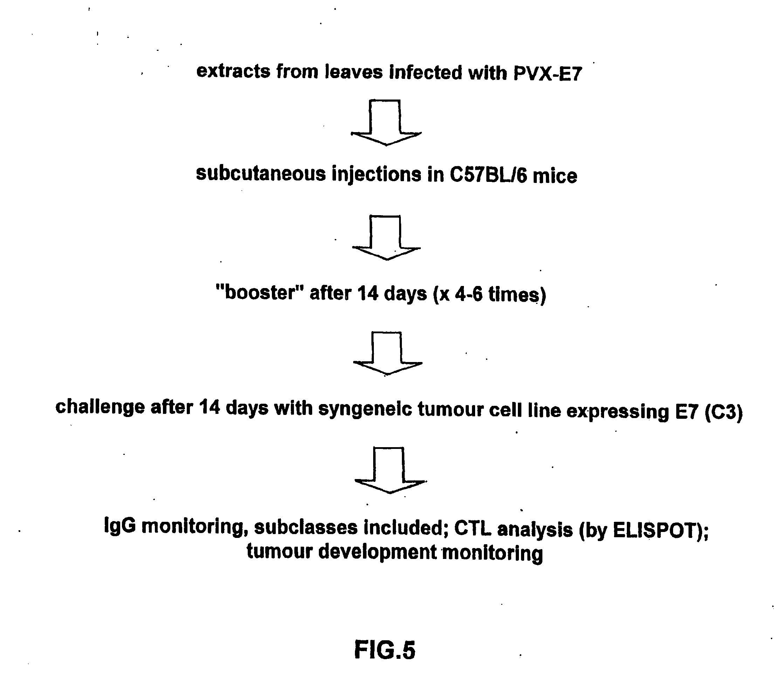

Figure 5 shows the scheme for an experimental vaccination in animal model (mouse).

### Detailed description of the invention

His-E7 protein, produced by E. coli and purified according to standard procedures, was added to plant extracts derived from *Nicotiana benthamiana* prepared according to the processes described hereinafter.

This mixture was administered to mice for vaccination purposes along with the preparation described hereinafter.

As it is reported in the summary of the invention, in a preferred embodiment antigen and adjuvanting extract are combined by a process of in-plant production of heterologous antigens through infection with vectors of viral origin (in plasmid form or as virions contained in plant juices). In particular, the gene encoding the E7 oncoprotein was amplified from a plasmid containing the genome of the human papillomavirus 16 (HPV16) by PCR (polymerase chain reaction) and cloned in a vector derived from the potato virus X (PVX) for the transient expression in-plant.

However, it should be highlighted that in the abovementioned process there may be used all the plant viruses belonging to the potexvirus genus, and in general all the vectors known to the art useful for the transfection of *Nicotiana benthamiana, Nicotiana tabacum* and *Chenopodium quinoa*, in particular any virus able to infect such a plant and potentially non-injurious to the subjects to be immunized (a limitation superfluous in case the vaccinogen should be purified by usual processes).

Viral vectors, by virtue of their features (multiplication rate, absence of pathogenicity in humans, stability at room temperature, etc.) are a particularly effective tool for the in-plant expression of heterologous proteins.

However, in order to obtain vaccinogen-containing extracts, also bacterial vectors (like, e.g. Agrobacterium tumefaciens) are useful, which can be employed both for transient expression by agroinfiltration, and to obtain stable expression through the production of transgenic plants. Likewise, physical and/or chemical transformation systems are useful (e.g., a biolistic system or by polyethylene glycol, PEG) with which not only the main nuclear genome but also that of the plastids (e.g., chloroplast) can be genetically transformed. These systems, alternative to the transformation of the nuclear genome, can greatly enhance the expression level of the heterologous protein.

Hence, the use of viral vectors and in particular of the PVX vector detailed hereinafter merely constitutes a particularly preferred embodiment.

Also the selection of the antigen produced in the model system (the E7 protein, whose gene was cloned starting from the codon encoding the first methionine, to produce a 98-aa protein) was performed, as this protein, by virtue of its features, is representative of the vaccinogens particularly suitable to be used in the processes and for the vaccine compositions of the invention.

Said protein is recognised as tumour antigen of the HPV-associated cervical carcinoma, and therefore as a putative vaccinogen for stimulating an immune response aimed at curing the tumour. Moreover, said protein is per se scarcely immunogenic and highly unstable and it requires the adding of adjuvants in order to induce a cell-mediated immune response (see state of the art above). Through the inventive process, the E7 protein results in an already adjuvated form enabling it to elicit a valid therapeutic response.

However, other antigens are likewise useful to the ends of the invention, in particular those having analogous features with respect to E7.

Hence, in particular the antigens useful in the process of the invention comprise viral and bacterial pathogenic proteins.

In fact, by the process of the invention it is possible to produce extracts comprising any one vaccinogen of protein nature of known nucleotide and/or amino acid sequence. Therefore, in particular there are comprised the pathogen-derived vaccinogens in general or associated to specific pathologies, like, e.g., viral or bacterial pathologies, or pathologies anyhow associated to the presence of microorganisms, and several tumour forms, (e.g., tumour-associated antigens) or pathologies caused by or anyhow associated to the presence of specific molecules.

Particularly suitable to be utilized in this procedure are viral antigens having well-known immunological features, and hence identifiable as putative vaccinogens. An exemplary virus is the human papillomavirus (HPV). In fact, HPV genome expresses only 8 proteins whose specific function is still not completely clear, including the abovementioned E7 protein.

The gene constructs of interest in the process of the invention generally comprise, besides all the functions required for PVX replication (genes 166, 25, 12 8 K and CP) a duplicated promoter for the gene of the virus-coat protein, so as to allow the expression of a soluble protein (see Figure 1).

Said constructs can comprise the gene of interest operatively linked to a signal sequence allowing to vehicle the protein in the secretory route and in the apoplast of the plant cell, and therefore to obtain a more concentrated and, possibly, stabler product.

Moreover, at the carboxy- or amino- terminal of the protein tags can advantageously be added which may be useful in troublesome native protein purifications.

Tags useful for said purpose are the c-myc tag and the flag tag, against which specific monoclonal antibodies for immune purification or a poly-histidine tail (His6) for purification by IMAC (Immobilized-metal affinity chromatography) are available.

Plasmid pPVX201-E7 proved particularly advantageous as a vector (Figure 1). This vector derives from plasmid pPVX201 (Baulcombe D. C., Chapman S., Santa Cruz S. (1995). 'Jellyfish green fluorescent protein as a reporter for virus infections'. *The Plant Journal* 7: 1045-1053), in turn derived from vector pGC3 (Chapman S., Kavanagh T., Baulcombe D. (1992). 'Potato virus X as a vector for gene expression in plants'. *The Plant Journal* 2: 549-557). The former contains unique sites for the cloning of the heterologous gene downstream of the duplicated promoter for the subgenomic RNA of the PVX coat protein, and it allows to infect plants directly with the plasmid DNA, since the entire PVX genome is controlled by the 35S promoter of the cauliflower mosaic virus (CaMV).

Concerning the plant host, as abovereported, the transfection was conducted in *Nicotiana* benthamiana, *Nicotiana tabacum* and *Chenopodium quinoa* plants.

Upon infection of *N. benthamiana* with pPVX201-E7, the symptoms of PVX infection generally appear 6-7 after inoculation, along with the expression of soluble E7 as vaccinogen of interest.

Significant amounts of E7 protein were detected in leaf extracts of plants infected with a vector pPVX201-E7 (for simplicity hereinafter referred to as PVX-E7), in particular both in the inoculated leaves and in the apical ones with symptoms of infection. This was assayed by Western blot, in which a mouse polyclonal antibody against the recombinant E7 protein produced in *E. coli* (His-E7), diluted 1:1000 was used for the detection (Figure 2), and by ELISA assay (Figure 3) in which a mouse polyclonal antibody against the E7-His protein, diluted 1:1000, was used for the detection. In particular the amount of E7 protein present in the extracts was accurately assessed by TAS-ELISA, using rabbit anti-E7 antibodies (see example 2).

No immunoreactive bands were detected in leaf extracts from plants infected with plasmid pPVX201 or PVX wild type virions, indicating the specificity of the preparation for the cloned antigen.

As evidence of the stability of the construct pPVX201-E7, high expression levels were detected in all plants at least until the fifth passage of infection with leaf extracts containing the recombinant virus.

The construct stability and the maintenance of the gene integrity, even after several reinfection passages, allowed an in-plant production of the E7 protein in an antigenically active form. This entails several advantages, e.g. the accumulation of the entire antigenic protein rather than a small epitope of it, as in the case of the display of protein portions at the surface of chimeric VLPs (virus like particles) in which regions able to induce a CTL response to E7 in mice are fused to the L1/L2 HPV proteins and used as vaccine of prophylactic/therapeutic type.

In particular, the plant-derived E7 proteins according to the process of the invention proved immunogenically similar in all to the E7-His protein derived from E. coli, with the advantage that in this case no purification was needed (Figure 4).

In particular, in experimental vaccination tests with PVX-E7 plant extracts, by subcutaneous administration and with no adjuvant addition, in animal models (mice) and subsequent challenge with a tumour line expressing E7, a protection from the tumour was obtained in about the 50% of the vaccinated mice (Figure 5).

The extracts can derive from foliar juices, upon extraction of the total soluble proteins with mild methods in absence of detergent and of denaturing substances, as well as in an enriched form from the apoplast, upon extraction of the intercellular fluids by vacuum infiltration.

Said tissues and/or the partial purification products thereof, like the intercellular fluids, can be used for purification and tests of biological activity in animals. Concerning the storing modes, for a short-time storage (e.g. of some days) the excised plants or leaves can be stored at room temperature or at + 4°C. The infected tissues (e.g., whole leaves) are still able to induce viral infection symptoms after a prolonged (more than one year) storing at -80°C. Alternatively, infected plant extracts can successfully yield new productive infections after storing at -20°C, even for relatively lengthy times (more than one month). The heterologous gene encoding the E7 protein is stably maintained during the infection stages of the virus and it seems not to interfere with its replication at least until the fifth reinfection passage, by mechanical infections, with infected juices. Also the expression level of the E7 protein in the various passages is unchanged, at estimated values of about 100-150 ng of protein per 100 µl of extract, obtained as disclosed in the examples. Hence, the genetic stability of the construct is matched by a likewise stable expression level.

Concerning the stability of the E7 protein, said protein is present in the extracts even after storing at + 4°C for several days (more than 15), as demonstrated by its reactivity in ELISA and in W. blot with a specific polyclonal serum.

The above crude extracts, administered to mice (several times, at intervals of about 15 days), in particular with 500 µl of extracts containing recombinant E7, as they are or with adjuvant (Quil-A), led to an effective immune response.

In particular, a certain number of immunized mice generated a specific CTL response, and even after 40 days were still tumour free.

80% of the vaccinated mice also developed an antibody response against the plant-produced E7 protein starting from the 4^{th} immunization, even in the absence of adjuvant, and this response proved higher than that produced by the same extracts in the presence of adjuvant. Then, in a preliminary testing, the mice 'vaccinated' with the PVX-E7 extracts were injected with cells of a tumour line containing the gene E7 of HPV16, demonstrating protection from tumour challenge in about the 50% of the animals.

Also the presence of delayed hypersensitivity, measured through ear thickness micrometering of vaccinated mice at 24, 48, and 72 hours from antigen inoculation, was' detected in a significant number of animals.

Hence, it was demonstrated that a vaccinogen can be expressed in plants at significant levels and that crude extracts can be used for vaccination purposes without purification and without adjuvants.

The preferred administration mode in the case of pathogens infecting the mucosal surfaces, like the HPV, is the oral one as the latter is particularly useful to induce both humoral and mucosal immunity (prophylactic vaccine).

For plant-produced HPV antigens, in particular for the E7 protein (therapeutic vaccine), the immunization scheme in model animals (mice) is based on the subcutaneous administration of several doses of antigen, starting from crude leaf extracts. Likewise, the vaccination could be carried out with partially purified extracts or with the purified protein. Moreover, the immunization scheme could envisage different vaccinogen doses and administration at different times. The effectiveness of the immunization with plant extracts, compared to that carried out with the E7-His protein, in the presence of Quil-A as adjuvant, effective in the rejection of the E7-expressing tumours, demonstrates these vaccinal schemes to be equipotent.

Moreover, other routes of administration like the subcutaneous and the mucosal one can also influence the immune response to the plant extracts. The injection scheme, the dose to be injected and the administration route which generate the strongest humoral response or the strongest cell-mediated immunity are those detailed hereinafter.

Hereto, merely a general outline of the present invention has been provided. With the aid of the following examples, hereinafter a more detailed description of specific embodiments thereof will be provided, aimed at making better understood its aims, features, advantages and operation modes.

### EXAMPLES

### Example 1: Construction of a plasmid for the PVX-mediated expression

The HPV 16-E7 gene was amplified by PCR from the plasmid E7-pGEX-4T1 (Di Lonardo A., Marcante M. L., Poggiali F., Venuti A. (1998). 'HPV16 E7 antibody levels in cervical cancer patients: before and after treatment'. *Journal of Medical Virology* 54: 192-195) using as primers the oligonucleotides E7 For (5'GGC CAT CGA TTC TAG AC **ATG** CAT GGA GAT ACA CCT ACA CAT TG 3', sites for the enzymes Cla I and Xba I underlined, codon for Methionine bolded) and E7 Rev (5' GGC CGT CGA CCC C GGG **TTA** TGG TTT CTG AGA ACA GAT GGG 3', sites for the enzymes Sal I and Sma I underlined, STOP codon bolded).

After digestion with the restriction enzymes Cla I and Sal I, the fragment was cloned in the vector pPVX201, obtaining the plasmid pPVX201-E7, which allows soluble expression of the E7 protein upon manual infection with the DNA plasmid (Figure 1).

After infection with DNA plasmid, *N. benthamiana* plants showing symptoms of infection were obtained 6-7 days post-inoculation. PVX-E7 extracts were also used for subsequent manual inoculation of fresh plants in order to study the stability of the construct.

### Example 2: Preparation of plant extract and analysis of expression of E7 protein

E7 expression was analysed both by Western blot and ELISA. Plant soluble proteins were obtained by homogenization of leaves in a blender with liquid nitrogen. The resulting powder was resuspended (0.3 g of fresh leaf /ml buffer) in extraction buffer 1xPBS, containing protease inhibitors ('Complete, EDTA-free', Boehringer Mannheim). The extract was centrifuged 10 min at 12.000 g and supernatant collected. The proteins present in the plant extracts were separated by 12% SDS-PAGE and transferred by electroblotting onto PVDF filters. The presence of E7 protein was detected by using a mouse polyclonal serum obtained against the His-E7 purified from E. coli, diluted 1:1000, and subsequently a goat anti-mouse IgG conjugated with HRP. Reactions were developed with the ECL system (Amersham) (Figure 2).

PVX-E7 leaf extracts were used in order to determine the amount of total soluble protein (TSP) by using the Bradford assay (BioRad) and bovine serum albumin was used as a protein standard. The amount of E7 protein in the extracts was estimated by a triple antibody sandwich (TAS)-ELISA. A rabbit polyclonal antibody against E7 was diluted 1:500 in carbonate coating buffer, pH 9.6, and used for coating the ELISA plates for 2 hours at room temperature. After blocking with 5% skimmed milk in PBS, 100 µl of leaf extracts were added and incubated overnight at 4°C, utilising known amounts of E7 purified from bacteria for comparison. Then, the samples were incubated with a mouse anti His-E7 polyclonal antibody, diluted 1:1000 and detection was done with a goat anti-mouse antibody conjugated to Horseradish peroxidase (HRP) (Figure 3).

### Example 3: Vaccine composition made of a plant extract to which the purified E7 protein is added.

The E7-His protein produced from *E. coli* and purified according to standard procedures (PVX-WT+E7) was added to plant extracts deriving from *N. benthamiana,* infected with the PVX wild type (PVX-WT), prepared according to the procedures described in example 2.

Systemic leaves showing typical symptoms of infection were subjected to total protein extraction in PBS buffer with and without protease inhibitors, according to the process described in example 2.

For a better characterisation, the foliar extracts of PVX-WT *N. benthamiana*, to which the purified His-E7 protein had been added from the outside at a final concentration of 8.3 ng/µl (PVX-WT+E7) in the extract, were kept at room temperature and at +4°C for 2 hours prior to analysis by Western blot and ELISA.

The results of the Western blot performed comparing the PVX-WT+E7 and the PVX-E7 extracts highlight the inexistence of significant differences between the two patterns. Moreover, the extracts exhibit a satisfactory and comparable ELISA activity.

Furthermore, no relevant difference was highlighted among the PVX-E7 patterns and the PVX-WT+E7 patterns obtained in the presence and in the absence of protease inhibitors. In the absence as well as in the presence of protease inhibitors both extracts exhibit a good ELISA reactivity (even after storing at +4°C for more than 25 days), whereas the inhibitor-free extracts anyhow exhibit a reactivity sensibly improved with respect to the inhibitor-provided extracts.

Moreover, the related patterns are not affected by the incubation temperature, given a 2 hour incubation time.

Accordingly, the outside addition of the vaccinogen, and in particular of the E7, in a PVX-WT-infected plant extract results in a composition having properties comparable to those of a composition in which the vaccinogen is internally produced, according to the hereto exemplified procedures.

### Example 4: Immunization of animals

PVX-E7-infected plant extracts (500 µl) and PVX-WT plant extracts (500 µl) to which E7-His protein produced from *E. coli* and purified (0.5 µg,) was added from the outside (PVX-WT+E7) were administered subcutaneously, with or without Quil-A as adjuvant, to C57BL/6 mice. Immunizations were repeated, at 15 days interval. As a control, several mice were immunized with the recombinant E7-His protein purified from *E. coli* plus Quil-A. 15 days after the final booster, animals were bled and the sera were analyzed for the presence of anti-E7 specific antibodies by ELISA. The results (Figure 4) show that the mice immunized with leaf extracts had developed anti-E7 antibodies, with no need of additional adjuvants.

### Example 5: Tumour challenge

15 days after the final booster, the 'vaccinated' mice were challenged subcutaneously with 4x10⁵ cells of a syngeneic tumour line expressing E7 (C3). The CTL response was tested by ELISPOT. Briefly: 96-well filtration plates were coated with anti-mouse IFN γ antibody and incubated overnight, in order to capture the IFN-γ secretion by these activated T cells. For detection of CD4 and CD8 cell precursors, splenocytes from vaccinated mice were harvested on day 14 after C3 challenge and added to the wells along with interleukin-2 (15 units/ml). Cells were incubated for 24 hours with or without peptides representing E7-specific H-2D^{b} CTL epitope (aa. 49-57). After culture, the plates were washed, incubated with biotinylated anti-mouse IFNγ antibody, washed again and incubated with avidin-alkaline phosphatase. The reaction between the IFN-γ and its antibody is highlighted by spots, developed with BCIP/NBT. Tumour mass in C3 challenged mice was measured too, in order to highlight a possible delay in the growth thereof.

### Example 6: Determination of E7-specific antibodies

The presence of E7-specific IgG in the sera of mice immunized with the recombinant plant E7 protein was assayed by ELISA. 96-well polystyrene plates were coated overnight at 4°C with the E7-His protein (1 µg/ml), diluted in carbonate buffer (50 mM NaHCO3, pH 9.6).

After blocking with a solution containing skimmed milk 5% in PBS for 2 hours, the sera of the immunized mice diluted 1:50 or 1:100 in a PBS 0.5% Tween-20 buffer were added to the samples.

Detection of total IgG and IgG2a subclass was performed by the respective biotin conjugate goat antimouse IgG. The signal was amplified by adding Horseradish peroxidase conjugate streptavidin (Southern Biotechnology Associates, Inc.). The reaction was developed by adding ABTS (2,2-azino-bis-benzthiazoline-6-sulfonic acid) solution (Southern Biotechnology Associates, Inc.). The optical density of the samples was determined at 405 nm using an ELISA reader.

### Example 7: Comparison of E7 expression in several plant systems infected with PVX-E7 virion.

In order to evaluate the validity of systems different from the *N. benthamiana, Chenopodium quinoa* (fam. Chenopodiaceae), *Nicotiana benthamiana*, and *N. tabacum* (fam. Solanaceae) plants were infected with an extract of leaf juices derived from *N. benthamiana* infected with the plasmid pPVX201-E7.

Then an immunoblotting (extraction in PBS buffer with the addition of protease inhibitors as abovedescribed) and an ELISA were performed on systemic leaves, or on inoculated leaves of each plant according to the abovedescribed process.

The result highlighted:
a. *Systemic leaves*
   - *N. benthamiana:* symptoms appeared 5-6 days after infection, in W. blot exhibiting the typical pattern hereto described and a good reactivity in in ACP (antigen coated plate) and in TAS-ELISA;
   - *N*. *tabacum:* symptoms appeared about 9 days after infection. The W. Blot pattern is in all similar to that of *N. benthamiana,* and the reactivity in ELISA is significant (triple with respect to the blank) though weaker than that exhibited by *N. benthamiana;*
   - *C. quinoa:* no symptoms, even one month after infection, along with an absence of signal for E7 in W. Blot and in ELISA;
*b. Inoculated leaves*
   - *N. benthamiana:* symptoms appeared about 7-8 days after the infection. Both the W. Blot and the ELISA results respectively exhibit a pattern and a reactivity that are similar, though weaker, with respect to those observed for the systemic leaves;
   - *N. tabacum:* symptoms appeared 7 days after infection. W. blot results highlight a pattern similar to that observed for the systemic leaves, with a band having the molecular weight of E7, though of reduced intensity. The reactivity in ELISA is significant, though weaker with respect to that of *N. benthamiana;*
   - *C. quinoa:* clear symptoms appeared 6 days after infection. W. blot results highlight a very strong pattern, with a more intense band at a molecular weight corresponding to that of E7, absent in the wild type PVX-infected plants. The reactivity in ELISA is high, comparable to that of *N. benthamiana.*

These results demonstrate the relevance of the three systems for the purposes of the invention.

To the abovedescribed processes, uses, vectors, vaccines and plants a person skilled in the art, in order to satisfy further and contingent needs, could effect several further modifications and variants, all however encompassed by the protection scope of the present invention, as defined by the appended claims.

### BIBLIOGRAPHICAL REFERENCES

- Baulcombe D. C., Chapman S., Santa Cruz S. (1995). 'Jellyfish green fluorescent protein as a reporter for virus infections'. *The Plant Journal* 7: 1045-1053.
- Breitburd F., Coursaget P. (1999). 'Human papillomavirus vaccines'. *Semin. Cancer Biol.* 9:431-444.
- Chapman S., Kavanagh T., Baulcombe D. (1992). 'Potato virus X as a vector for gene expression in plants'. *The Plant Journal* 2: 549-557.
- Di Lonardo A., Marcante M. L., Poggiali F., Venuti A. (1998). 'HPV16 E7 antibody levels in cervical cancer patients: before and after treatment'. *Journal of Medical Virology* 54: 192-195.
- Fernando G. J. P., Murray B., Zhou J., Frazer I. H. (1999). 'Expression, purification and immunological characterisation of the transforming protein E7, from cervical cancer-associated human papillomavirus type 16'. *Clin. Exp. Immunol.* 115: 397-403.
- Franconi R., Roggero P., Pirazzi P., Arias F. J., Desiderio A., Bitti O., Pashkoulov D., Mattei B., Bracci L., Masenga V., Milne R. G., Benvenuto E. (1999). 'Functional expression in bacteria and in plants of an scFv antibody fragment against tospoviruses'. *Immunotechnology* 4: 189-201.
- Gupta, R. K. (1998). 'Aluminium compounds as vaccine'. *Advanced Drug Delivery Reviews* **32:** 155-172.
- Hansson M., Nygren P.-A., Stahl S. (2000). 'Design and production of recombinant subunit vaccines' *Biotechnol. Appl. Biochem.* 32: 95-107.
- Hongxiu et al. (1999). *Human gene therapy* 10: 2727-2740.
- Singh M., O'Hagan D. (1999). 'Advances in vaccine adjuvants'. *Nature Biotechnology* 17: 1075-1081.
- Reinstein E, Scheffner M, Oren M et al (2000). 'Degradation of the E7 human papillomavirus oncoprotein by the ubiquitin-proteasome system: targeting via ubiquitination of the N-terminal residue'. *Oncogene* 19: 5944-5950.

## Claims

1. Use of an extract in buffer without detergent and denaturing substances of total soluble proteins from leaf cells of a plant selected from the group comprising *Nicotiana benthamiana, Nicotiana tabacum* and *Chenopodium quinoa,* for the preparation of an adjuvant composition to be used with a subunit vaccines comprising a vaccinogen of interest.

2. Use according to claim 1, wherein said plant is *Nicotiana benthamiana.*

3. Use according to claim 1, wherein said plant is infected with a plant vector expressing in leaf cells the vaccinogen of interest.

4. Use according to claim 3, wherein said plant vector is a plant viral vector.

5. Use according to claim 4, wherein the plant viral vector is derived from potato virus X (PVX).

6. Use according to claim 3, wherein said vaccinogen of interest is a viral antigen.

7. Use according to claim 3, wherein the vaccinogen of interest is the E7 protein of the human papillomavirus (HPV).

8. Vaccine comprising a vaccinogen of interest and as adjuvant an extract in buffer without detergent and denaturing substances of total soluble proteins from leaf cells of a plant selected from the group comprising *Nicotiana benthamiana, Nicotiana tabacum* and *Chenopodium quinoa.*

9. Vaccine according to claim 8, wherein said plant is *Nicotiana benthamiana.*

10. Vaccine according to claim 8 or 9, wherein said vaccinogen is a viral antigen.

11. Vaccine according to any one of claims 8 to 10, wherein said vaccinogen is the E7 protein of the human papillomavirus (HPV).

12. Vaccine according to any one of claims 8 to 11, obtainable by a process comprising the steps of:
a. expressing in soluble form a protein antigen in leaf cells of the plant infected by a plant vector expressing said antigen; and
b. preparing an extract of total soluble proteins from the cells of foliar tissues with symptoms of infection, to which a high expression level of the heterologous protein is associated;
said plant extract being useful as subunit vaccine.

13. Vaccine according to claim 12, wherein the plant vector is a viral vector.

14. Vaccine according to claim 13, wherein said viral vector is derived from potato virus X (PVX).

15. Vaccine according to any one of claims 12 to 14, wherein step b. is carried out using extraction techniques of total soluble proteins such that said extraction is performed in the absence of detergents and of denaturing substances.

16. Process for the production of a subunit vaccine comprising the steps of:
a. transiently expressing in soluble form a protein antigen in leaf cells of a plant selected from the group comprising *Nicotiana benthamiana, Nicotiana tabacum* and *Chenopodium quinoa,* infected by a plant vector expressing said antigen; and
b. preparing an extract in buffer without detergent and denaturing substances of total soluble proteins from the cells of foliar tissues with symptoms of infection, to which a high expression level of the heterologous protein is associated;
said plant extract of total soluble proteins being used as subunit vaccine.

17. Process according to claim 16, wherein said plant is *Nicotiana benthamiana.*

18. Process according to claim 16 or 17, wherein said antigen is a viral antigen.

19. Process according to claim 18, wherein said antigen is the E7 protein of the human papillomavirus (HPV).

20. Process according to any one of claims 16 to 19, wherein the plant vector is a plant viral vector.

21. Process according to claim 20, wherein said plant viral vector is derived from potato virus X (PVX).

22. Kit for the treatment of pathologies for the therapy of which it be necessary or advisable to induce an immune response of humoral as well as of cell-mediated type towards a determined vaccinogen comprising
- a composition comprising said vaccinogen along with compatible additives; and
- an extract in buffer without detergent and denaturing substances of total soluble proteins from leaf cells of a plant selected from the group comprising *Nicotiana benthamiana, Nicotiana tabacum* and *Chenopodium quinoa;*
for concomitant, sequential or separated use in the treatment of said pathologies.

23. Kit according to claim 22, wherein said plant is *Nicotiana benthamiana.*

## Patentansprüche

1. Verwendung eines Extraktes der gesamten löslichen Proteine aus Blattzellen einer Pflanze, ausgewählt aus der Gruppe umfassend *Nicotiana benthamiana, Nicotiana tabacum* und *Chenopodium quinoa* in Puffer ohne Detergens und denaturierende Stoffe für die Herstellung einer Adjuvanszusammensetzung zur Verwendung mit Untereinheiten-Impfstoffen, umfassend ein interessierendes Vakzinogen.

2. Verwendung nach Anspruch 1, wobei die Pflanze *Nicotiana benthamiana* ist.

3. Verwendung nach Anspruch 1, wobei die Pflanze mit einem Pflanzenvektor infiziert wird, der das interessierende Vakzinogen in Blattzellen exprimiert.

4. Verwendung nach Anspruch 3, wobei der Pflanzenvektor ein viraler Pflanzenvektor ist.

5. Verwendung nach Anspruch 4, wobei der virale Pflanzenvektor vom Kartoffelvirus X (PVX) abgeleitet ist.

6. Verwendung nach Anspruch 3, wobei das interessierende Vakzinogen ein virales Antigen ist.

7. Verwendung nach Anspruch 3, wobei das interessierende Vakzinogen das E7-Protein des menschlichen Papillomavirus (HPV) ist.

8. Impfstoff, umfassend ein interessierendes Vakzinogen und als Adjuvans einen Extrakt der gesamten löslichen Proteine aus Blattzellen einer Pflanze, ausgewählt aus der Gruppe umfassend *Nicotiana benthamiana, Nicotiana tabacum* und *Chenopodium quinoa* in Puffer ohne Detergens und denaturierende Stoffe.

9. Impfstoff nach Anspruch 8, wobei die Pflanze *Nicotiana benthamiana* ist.

10. Impfstoff nach Anspruch 8 oder 9, wobei das Vakzinogen ein virales Antigen ist.

11. Impfstoff nach einem der Ansprüche 8 bis 10, wobei das Vakzinogen das E7-Protein des menschlichen Papillomavirus (HPV) ist.

12. Impfstoff nach einem der Ansprüche 8 bis 11, erhältlich durch ein Verfahren umfassend die Schritte
(a) Exprimieren eines Proteinantigens in löslicher Form in Blattzellen der Pflanze, die mit einem Pflanzenvektor infiziert ist, der das Antigen exprimiert; und
(b) Herstellen eines Extraktes der gesamten löslichen Proteine aus den Zellen von Blattgeweben mit den Symptomen einer Infektion, mit denen ein hohes Expressionsniveau des heterologen Proteins assoziiert ist;
wobei der Pflanzenextrakt nützlich als Untereinheiten-Impfstoff ist.

13. Impfstoff nach Anspruch 12, wobei der Pflanzenvektor ein viraler Vektor ist.

14. Impfstoff nach Anspruch 13, wobei der virale Vektor vom Kartoffelvirus X (PVX) abgeleitet ist.

15. Impfstoff nach einem der Ansprüche 12 bis 14, wobei Schritt (b) unter Verwendung von Extraktionstechniken der gesamten löslichen Proteine in der Weise ausgeführt wird, dass die Extraktion in Abwesenheit von Detergenzien und denaturierenden Stoffen durchgeführt wird.

16. Verfahren zur Herstellung eines Untereinheiten-Impfstoffs, umfassend die Schritte:
(a) transientes Exprimieren eines Proteinantigens in löslicher Form in Blattzellen einer Pflanze, ausgewählt aus der Gruppe umfassend *Nicotiana benthamiana, Nicotiana tabacum* und *Chenopodium quinoa,* die mit einem Pflanzenvektor infiziert wurde, der das Antigen exprimiert; und
(b) Herstellen eines Extraktes der gesamtem löslichen Proteine in Puffer ohne Detergens und denaturierende Stoffe aus den Zellen von Blattgeweben mit Symptomen einer Infektion, mit denen ein hohes Expressionsniveau des heterologen Proteins assoziiert ist;
wobei der Pflanzenextrakt der gesamten löslichen Proteine als Untereinheiten-Impfstoff verwendet wird.

17. Verfahren nach Anspruch 16, wobei die Pflanze *Nicotiana benthamiana* ist.

18. Verfahren nach Anspruch 16 oder 17, wobei das Antigen ein virales Antigen ist.

19. Verfahren nach Anspruch 18, wobei das Antigen das E7-Protein des menschlichen Papillomavirus (HPV) ist.

20. Verfahren nach einem der Ansprüche 16 bis 19, wobei der Pflanzenvektor ein viraler Pflanzenvektor ist.

21. Verfahren nach Anspruch 20, wobei der virale Pflanzenvektor vom Kartoffelvirus X (PVX) abgeleitet ist.

22. Kit zur Behandlung von pathologischen Befunden, zu deren Therapie es notwendig oder ratsam ist, eine Immunantwort vom humoralen sowie vom zellvermittelten Typ gegen ein bestimmtes Vakzinogen zu induzieren, umfassend
- eine Zusammensetzung umfassend das Vakzinogen zusammen mit kompatiblen Additiven; und
- einen Extrakt der gesamten löslichen Proteine aus Blattzellen einer Pflanze, ausgewählt aus der Gruppe umfassend *Nicotiana benthamiana, Nicotiana tabacum* und *Chenopodium quinoa* in Puffer ohne Detergens und denaturierende Stoffe;
zur gleichzeitigen, sequenziellen oder getrennten Verwendung in der Behandlung dieser pathologischen Befunde.

23. Kit nach Anspruch 22, wobei die Pflanze *Nicotiana benthamiana* ist.

## Revendications

1. Utilisation d'un extrait dans un tampon sans détergent ni substances dénaturantes des protéines solubles totales de cellules de feuilles d'un végétal choisi parmi le groupe comprenant *Nicotiana benthamiana, Nicotiana tabacum,* et *Chenopodium quinoa,* pour la préparation d'une composition d'adjuvant destinée à être utilisée avec des vaccins à virus fractionné comprenant un vaccinogène d'intérêt.

2. Utilisation selon la revendication 1, dans laquelle ledit végétal est *Nicotiana benthamiana.*

3. Utilisation selon la revendication 1, dans laquelle ledit végétal est infecté avec un vecteur végétal exprimant dans les cellules de feuilles le vaccinogène d'intérêt.

4. Utilisation selon la revendication 3, dans laquelle ledit vecteur végétal est un vecteur viral végétal.

5. Utilisation selon la revendication 4, dans laquelle le vecteur viral végétal est dérivé du virus X de la pomme de terre (PVX).

6. Utilisation selon la revendication 3, dans laquelle ledit vaccinogène d'intérêt est un antigène viral.

7. Utilisation selon la revendication 3, dans laquelle ledit vaccinogène d'intérêt est la protéine E7 du papillomavirus humain (PVH).

8. Vaccin comprenant un vaccinogène d'intérêt et en tant qu'adjuvant un extrait dans un tampon sans détergent ni substances dénaturantes des protéines solubles totales de cellules de feuilles d'un végétal choisi parmi le groupe comprenant *Nicotiana benthamiana, Nicotiana tabacum,* et *Chenopodium quinoa.*

9. Vaccin selon la revendication 8, dans lequel ledit végétal est *Nicotiana benthamiana.*

10. Vaccin selon la revendication 8 ou 9, dans lequel ledit vaccinogène est un antigène viral.

11. Vaccin selon l'une quelconque des revendications 8 à 10, dans lequel ledit vaccinogène est la protéine E7 du papillomavirus humain (PVH).

12. Vaccin selon l'une quelconque des revendications 8 à 11, pouvant être obtenu par un procédé comprenant les étapes consistant à :
a. exprimer sous une forme soluble un antigène protéique dans les cellules de feuilles du végétal infecté par un vecteur végétal exprimant ledit antigène ; et
b. préparer un extrait des protéines solubles totales issues des cellules des tissus foliaires présentant les symptômes d'infection, auquel un niveau d'expression élevé de la protéine hétérologue est associé ;
ledit extrait végétal étant utile en tant que vaccin à virus fractionné.

13. Vaccin selon la revendication 12, dans lequel le vecteur végétal est un vecteur viral.

14. Vaccin selon la revendication 13, dans lequel ledit vecteur viral est dérivé du virus X de la pomme de terre (PVX).

15. Vaccin selon l'une quelconque des revendications 12 à 14, dans lequel l'étape b. est mise en oeuvre en utilisant des techniques d'extraction des protéines solubles totales de telle manière que ladite extraction est effectuée en l'absence de détergents et de substances dénaturantes.

16. Procédé pour la production d'un vaccin à virus fractionné comprenant les étapes consistant à :
a. exprimer de façon transitoire sous une forme soluble un antigène protéique dans des cellules de feuilles d'un végétal choisi parmi le groupe comprenant *Nicotiana benthamiana, Nicotiana tabacum,* et *Chenopodium quinoa,* infecté par un vecteur végétal exprimant ledit antigène ; et
b. préparer un extrait dans un tampon sans détergent ni substances dénaturantes des protéines solubles totales issues de cellules des tissus foliaires présentant les symptômes de l'infection, auquel un niveau d'expression élevée de la protéine hétérologue est associé ;
ledit extrait végétal des protéines solubles totales étant utilisé comme vaccin à virus fractionné.

17. Procédé selon la revendication 16, dans lequel ledit végétal est *Nicotiana benthamiana.*

18. Procédé selon la revendication 16 ou 17, dans lequel ledit antigène est un antigène viral.

19. Procédé selon la revendication 18, dans lequel ledit antigène est la protéine E7 du papillomavirus humain (PVH).

20. Procédé selon l'une quelconque des revendications 16 à 19, dans lequel le vecteur végétal est un vecteur viral végétal.

21. Procédé selon la revendication 20, dans lequel ledit vecteur viral végétal est dérivé du virus X de la pomme de terre (PVX).

22. Kit pour le traitement de pathologies pour la thérapie desquelles il est nécessaire ou conseillé d'induire une réponse immunitaire de type humoral ainsi que de type médiatisé par des cellules envers un vaccinogène déterminé comprenant
- une composition comprenant ledit vaccinogène ainsi que des additifs compatibles ; et
- un extrait dans un tampon sans détergent ni substances dénaturantes des protéines solubles totales de cellules de feuilles d'un végétal choisi parmi le groupe comprenant *Nicotiana benthamiana, Nicotiana tabacum,* et *Chenopodium quinoa ;*
pour une utilisation concomitante, séquentielle ou séparée dans le traitement desdites pathologies.

23. Kit selon la revendication 22, dans lequel ledit végétal est *Nicotiana benthamiana.*
